(19) Europäisches Patentamt
European Patent Office
Office européen des brevets

(11) Veröffentlichungsnummer: **0 208 999**
**A1**

(12) **EUROPÄISCHE PATENTANMELDUNG**

(21) Anmeldenummer: 86109031.4

(22) Anmeldetag: 02.07.86

(51) Int. Cl.⁴: **C 07 C 103/58**, C 07 C 83/10, C 07 C 121/43, C 07 C 143/78, C 07 C 147/107, C 07 C 147/14, C 07 C 149/41, C 07 D 211/34, C 07 D 211/44, C 07 D 211/70, C 07 D 207/20

(30) Priorität: 18.07.85 DE 3525623

(43) Veröffentlichungstag der Anmeldung: 21.01.87 Patentblatt 87/4

(84) Benannte Vertragsstaaten: AT BE CH DE FR GB IT LI LU NL SE

(71) Anmelder: CELAMERCK GmbH & Co. KG, Binger Strasse 173, D-6507 Ingelheim am Rhein (DE)

(72) Erfinder: Curtze, Jürgen, Dr., Rheingaublick 6, D-6222 Geisenheim-Johannisberg (DE)
Erfinder: Albert, Guido, Dr. Dipl.-Biologe, Volxheimer Strasse 4, D-6551 Hackenheim (DE)
Erfinder: Drandarevski, Christo, Dr., Boehringer Strasse 8, D-6507 Ingelheim am Rhein (DE)
Erfinder: Pieper, Helmut, Dr. Dipl. Chem., Kapellenweg 5, D-7950 Biberach an der Riss (DE)
Erfinder: Nickl, Josef, Dr. Dipl.-Chem., Silcherstrasse 8, D-7950 Biberach an der Riss (DE)

(54) **Fungizid wirksame Acrylsäureamide.**

(57) Die Erfindung betrifft neue Acrylsäureamide der Formel

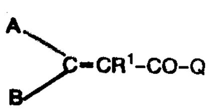

in der A, B, $R^1$ und Q wie im Text definiert sind, Verfahren zu deren Herstellung sowie fungizide Mittel gekennzeichnet durch einen Gehalt an Verbindungen der Formel (I).

EP 0 208 999 A1

Die Erfindung betrifft neue Acrylsäureamide der Formel:

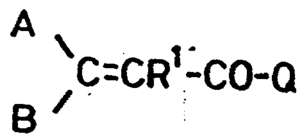

(I)

in der

$R^1$ für Wasserstoff, Halogen, Cyano, gegebenenfalls substituiertes Alkyl oder gegebenenfalls substituiertes Alkoxyalkyl steht

A für einen bis zu dreifach durch $R^2$ substituierten Phenylrest steht, wobei im Falle doppelter oder dreifacher Substitution $R^2$ unabhängig voneinander gewählt werden kann

B für

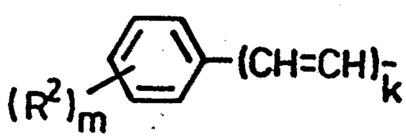

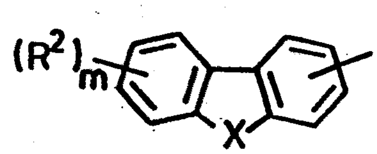

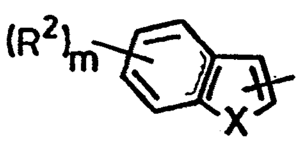

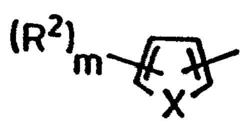

k für 0,1,2 steht

m für 0,1,2,3 steht, wobei $R^2$ unabhängig voneinander gewählt werden kann.

X für $CH_2$, O, S, NH oder N-Alkyl steht

$R^2$ für Halogen, Nitro, Hydroxy, Cyano, Carboxy, Alkoxycarbonyl, Carbamoyl, gegebenenfalls substituiertes Alkyl, gegebenenfalls substituiertes Alkoxy, gegebenenfalls substituiertes Alkyl-$S(O)_p$- mit p = 0,1,2, gegebenenfalls substituiertes Phenyl, gegebenenfalls substituiertes Phenoxy, Amino, Monoalkylamino, Dialkylamino, gegebenenfalls substituiertes Phenyl-$S(O)_p$ mit p = 0,1,2, gegebenenfalls substituiertes $C_3$-$C_7$-Cycloalkyl steht

Q für 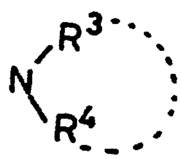 oder 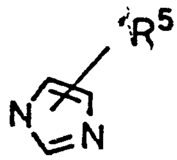

steht

$R^3$ für Wasserstoff gegebenenfalls substituiertes Alkyl, gegebenenfalls substituiertes Phenyl steht

$R^4$ für substituiertes Alkyl, substituiertes $C_3$-$C_7$-Cycloalkyl, substituiertes Alkenyl, substituiertes Phenyl, gegebenenfalls substituiertes Alkinyl, gegebenenfalls substituiertes Alkoxy, gegebenenfalls substituiertes $C_5$-$C_{18}$-Alkyl

$(CH_2)_{(q+1)}$-$^6$ oder (CH)-$R^8$-$(CH_2)_q$-CO-$R^7$ (mit q = 0,1,2) steht

$R^6$ für gegebenenfalls substituiertes Alkoxy,
für gegebenenfalls substituiertes Alkylthio,
für gegebenenfalls substituiertes Alkylamino,
für gegebenenfalls substituiertes Dialkylamino,
für gegebenenfalls substituiertes Morpholino,
für gegebenenfalls substituiertes $C_3$-$C_7$-Cycloalkyl,
für gegebenenfalls substituiertes $C_6$-$C_{10}$-Alkyl,
für gegebenenfalls substituiertes Phenyl,
für gegebenenfalls substituiertes Dialkoxymethyl,
für gegebenenfalls substituiertes Dialkylthiomethyl,
für gegebenenfalls substituiertes 1,3-Dioxolan-2-yl,

für Halogen, Hydroxy, Amino, Mercapto steht,

$R^7$ für Wasserstoff, Hydroxy, gegebenenfalls substituiertes Alkyl, Amino, Monoalkylamino, Dialkylamino, Alkoxy, gegebenenfalls substituiertes Morpholino, gegebenenfalls substituiertes Piperazino, gegebenenfalls substituiertes Imidazo, gegebenenfalls substituiertes Piperidino steht

$R^8$ für Wasserstoff, gegebenenfalls substituiertes Alkyl steht,

$R^3$ und $R^4$ gemeinsam für eine ggf. ungesättigte, ggf. benzokondensierte $C_3$-$C_5$-Alkylenkette, die durch O, NH, N-Alkyl, $S(O)_p$ mit $p = 0,1,2$ unterbrochen sein kann und ein oder mehrfach mit Halogen, Cyano, Nitro, Oxo, Hydroxy, Alkoxycarbonyl, ggf. substituiertem Carbamoyl oder Mercapto substituiert ist, stehen.

$R^5$ für einen an ein beliebiges Kohlenstoffatom des Imidazol gebundenen Rest aus der Gruppe Wasserstoff, oder gegebenenfalls substituiertes Alkyl steht,

sowie gegebenenfalls die Salze der vorstehend definierten Verbindungen mit Säuren, Basen oder Komplexbildern.

Im Rahmen der vorstehenden Definitionen können die Reste und Gruppen jeweils gleich oder verschieden sein.

Mit Alkyl sind $C_1$-$C_6$-Alkylradikale die geradkettig oder verzweigt sein können gemeint, bevorzugt sind $C_1$-$C_4$-Alkyle wie Methyl, Ethyl, n-Propyl, iso-Propyl, n-Butyl, sek-Butyl, tert-Butyl oder Isobutyl.

Die vorstehende Definition gilt auch wenn das Alkylradikal substituiert und/oder Bestandteil einer Alkoxyalkyl-, Alkoxycarbonyl-, Carbamoyl-, Alkoxy-, Alkylthio-, Alkylsulfinyl-, Alkylsulfonyl-, Monoalkylamino-, Dialkoxymethyl-, Dialkylthiomethyl-, Dialkylaminogruppe ist oder das Alkylradikal als Substituent an ein aromatisches, heterocyclisches oder carbocyclisches System gebunden ist.

Unter substituiertem Alkyl sind Alkylradikale zu verstehen, die ein oder mehrfach mit Hydroxy, Alkoxy, Mercapto, Halogen, Alkylthio, Nitro, Cyano oder Amino substituiert sind. Bevorzugt sind Halogen, Hydroxy, Cyano; hervorzuheben ist die Trifluormethyl und die Trichlormethylgruppe.

Im Falle der Carbamoylgruppen ist die unsubstituierte Carbamoylgruppe sowie die N,N-Dimethylcarbamoylgruppe bevorzugt.

Halogene sind Fluor, Chlor, Brom und Jod, vorzugsweise Fluor, Chlor und Brom und in zweiter Linie Jod.

Die Substituenten in den für A und B angegebenen Resten sind insbesondere Halogen, Nitro, Amino, gegebenenfalls ein- oder mehrfach halogensubstituierte $C_1$-$C_4$-Alkyl- und -Alkoxygruppen, NH($C_1$-$C_4$-Alkyl) und N($C_1$-$C_4$-Alkyl$_2$, auch Phenoxy, Phenylthio, $C_1$-$C_4$-Alkylthio.

Der Rest A ist bevorzugt di- oder tri-substituiert, wobei zwei Substituenten, z.B. Methyl, Methoxy, Ethyl, Ethoxy, Fluor, Chlor, Brom, $CHF_2O$, $CF_3$,$CF_2Cl$, $CF_3O$, $CH_3S$, $CH_3SO$, $CH_3SO_2$, $NH_2$, $NHCH_3$, $N(CH_3)_2$, sich bevorzugtin 3,4-Stellung befinden.

Sind A und B in der Formel I verschieden, so können die Verbindungen der Formel I als cis-/trans-Isomere vorliegen. Die Formel I umfaßt in diesem Fall sowohl die einzelnen Isomeren als auch Gemische der cis- und der trans-Verbindung.
Des weiteren können die Reste A bzw. B in ihrer freien Drehbarkeit um die Achse der Einfachbindung aufgrund sterischer oder sonstiger Sekundärwechselwirkungen beeinträchtigt sein; derartige Effekte können Atropisomerie hervorufen. Die Erfindung umfaßt somit auch die atropisomeren Strukturen von (I).
Der Substituent Q umfaßt offenkettige Amidstrukturen sowie die in der Definition angegebenen heterocyclischen Strukturen.
Im Falle Q = Imidazol sind Verbindungen bevorzugt bei denen $R^1$ von Wasserstoff verschieden ist.

Man erhält die neuen Verbindungen nach an sich bekannten Verfahren durch:

a) Umsetzung einer Acrylsäure der Formel

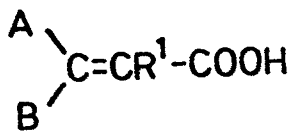

(II)

worin A, B und $R^1$ wie zuvor definiert sind oder durch Umsetzung eines gegebenenfalls in situ hergestellten reaktionsfähigen Derivates von (II) mit einer Verbindung der Formel

$$HQ \qquad (III)$$

in der Q wie zuvor definiert ist.
Das Verfahren stellt somit die Acylierung einer Verbindung der Formel III mit einer Carbonsäure der Formel II dar, wobei die Umsetzung vorteilhaft in Gegenwart eines die Säure II aktivierenden oder eines wasserentziehenden Mittels oder aber mit reaktiven Derivaten der Carbonsäure II oder des Edukts III gearbeitet wird.

Als gegebenenfalls im Reaktionsgemisch hergestellte reaktive Derivate einer Carbonsäure der Formel II kommen beispielsweise ihre Alkyl-, Aryl-, Aralkylester oder -thioester wie der Methyl-, Ethyl-, Phenyl- oder Benzylester, ihre Imidazolide, ihre Säurehalogenide wie das Säurechlorid oder -bromid, ihre Anhydride, ihre gemischten Anhydride mit aliphatischen oder aromatischen Carbon-, Sulfen-, Sulfin-, Sulfonsäuren oder mit Kohlensäureestern, z.B. mit der Essigsäure, der Propionsäure, der p-Toluolsulfonsäure oder der O-Ethylkohlensäure, oder ihre N-Hydroxyimidester in Betracht. Als gegebenenfalls im Reaktionsgemisch hergestellte reaktive Derivate eines Amins der Formel III eignen sich z.B. ihre "Phosphorazoderivate".

Als säureaktivierende und/oder wasserentziehende Mittel kommen beispielsweise ein Chlorameisensäureester wie Chlorameisensäureäthylester, Phosphorpentoxid, N,N'-Dicyclohexylcarbodiimid, N,N'-Carbonyldiimidazol oder N,N'-Thionyldiimidazol in Betracht.

Die Umsetzung wird zweckmäßigerweise in einem Lösungsmittel oder Lösungsmittelgemisch wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart einer anorganischen Base wie Natriumcarbonat oder einer tertiären organischen Base wie Triethylamin oder Pyridin, welche gleichzeitig als Lösungsmittel dienen kann, und gegebenenfalls in Gegenwart eines säureaktivierenden Mittels bei Temperaturen zwischen -25°C und 150°C, vorzugsweise jedoch bei Temperaturen zwischen -10°C und der Siedetemperatur des Reaktionsgemisches, durchgeführt. Hierbei braucht ein gegebenenfalls im Reaktionsgemisch entstandenes reaktionsfähiges Derivat einer Verbindung der allgemeinen Formeln II oder III nicht isoliert zu werden, ferner kann die Umsetzung auch in einem Überschuß der eingesetzten Verbindung der allgemeinen Formel III als Lösungsmittel durchgeführt werden.
Erfindungsgemäß erhaltene cis-/trans-Isomerengemische können gewünschtenfalls anschließend nach üblichen Methoden in die entsprechenden cis- und trans-Isomeren aufgetrennt werden. Das gleiche gilt für eventuelle Atropisomere.

b) Im Falle der Substituentenbedeutung Q = Imidazolyl ist es zweckmäßig die freie Carbonsäure II mit einem gewünschtenfalls entsprechend substituierten Carbonyldiimidazol als reaktive Form des Edukts III zum gewüschten Acrylsäureimidazolid der Formel I direkt umzusetzen.

c) Verbindungen der Formel (I) sind auch darstellbar durch Umsetzung eines Ketons der Formel (IV) mit einem Posphonoessigsäurederivat der Formel (XIV) in der R' vorzugsweise für einen niederen Alkylrest steht, nach dem verfahren von Wittig und Horner

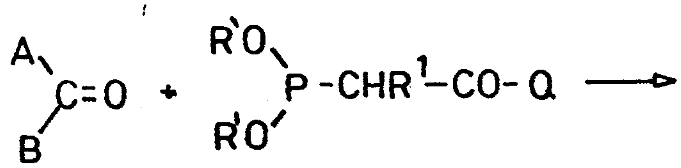

(I)

(IV) (XIV)

Die Isomerentrennung erfolgt vorzugsweise durch fraktionierte Kristallisation aus Methanol, Ethanol, Isopropanol, Methanol/Wasser oder Ethanol/Petrolether.

Verbindungen der Formel I mit basischen Gruppen können gewünschtenfalls in Säureadditionssalze übergeführt werden, vorzugsweise in Salze von Mineralsäuren wie Salzsäure, Bromwasserstoffsäure, Schwefelsäure, Phosphorsäure.

Die Acrylsäurederivate der Formel II sind bekannt oder können nach an sich bekannten Verfahren hergestellt werden.

Ausgangsstoffe der Formel II in denen die Reste A und B der Definition von Anspruch 1 entsprechen können ausgehend vom Keton der Formel IV nach zahlreichen an sich bekannten Verfahren hergestellt werden.

Durch Umsetzung von IV mit α-Halogencarbonsäureester V und anschließender Verseifung nach Reformatsky:

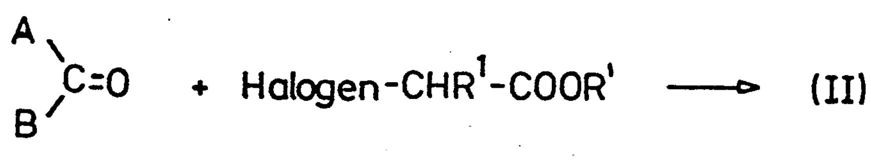

(IV) (V)

Durch Umsetzung von IV mit CH-aciden Komponenten nach Knoevenagel, hier erläutert anhand der Umsetzung von IV mit einem Nitril VI und anschließender Verseifung des Acrylnitrils VII zur Carbonsäure II.

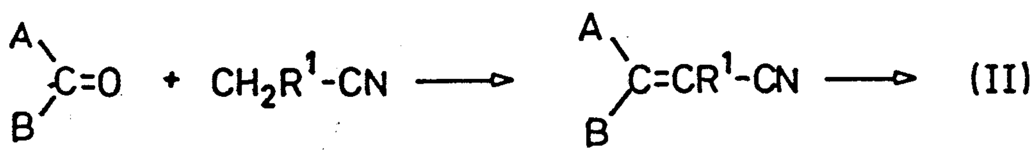

(IV) (VI) (VII)

Acrylsäuren der Formel II können auch nach Wittig-Horner ausgehend von Keton IV durch Umsetzung mit einer Phosphonessigsäureverbindung der Formel VIII und anschließender Verseifung des Esters IX hergestellt werden.

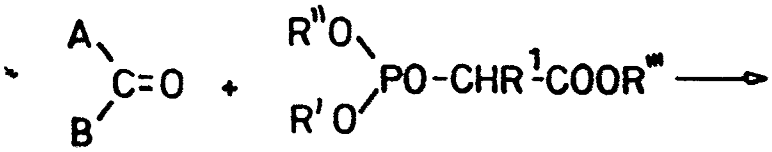

(IV) (VIII)

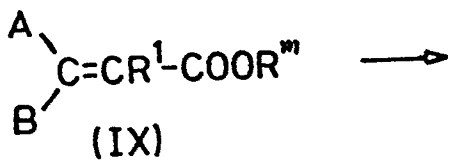

(II)

(IX)

Verbindungen der Formel II, in denen B für den Rest

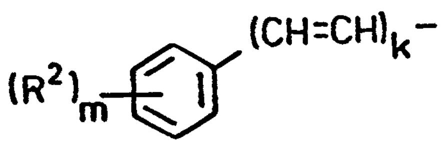

steht und k von null verschieden ist können ebenfalls nach Horner Wittig ausgehend von dem Acetophenon X über den Methacrylsäureester XI durch Kondensation mit der Carbonylkomponente XII zu XIII und anschließende Verseifung zu II in einer mehrstufigen Reaktionsfolge hergestellt werden:

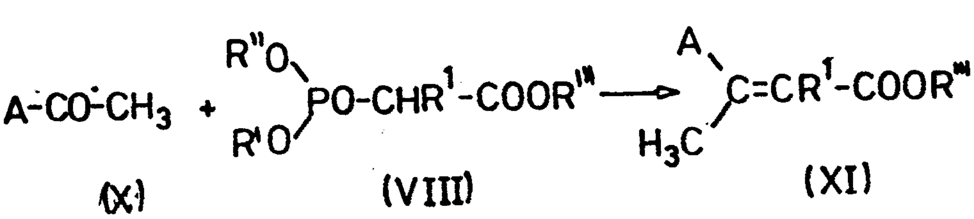

(X) (VIII) (XI)

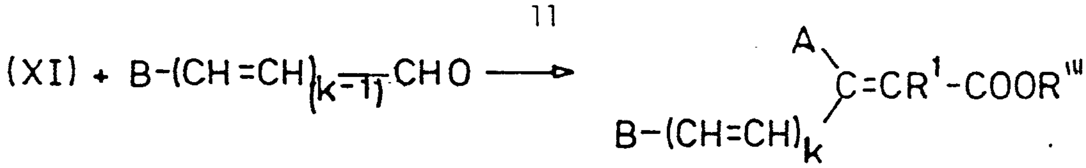

(XII) (XIII)

(XIII) $\longrightarrow$ (II)

Die Reste A, $R^1$, $R^2$, B und k haben die obige Bedeutung R', R" und R"' stehen vorzugsweise für niedere Alkylreste.

Die erfindungsgemäßen Verbindungen zeigen eine starke Wirkung besonders gegen phytopathogene Pilze, vor allem gegen echten Mehltau, falschen Mehltau (etwa Plasmopara und Phytophthora), Schorf, Grauschimmel, Rostpilze. Wegen ihrer nur sehr geringen Phytotoxizität können die neuen Verbindungen in praktisch allen Nutz- und Zierpflanzenkulturen eingesetzt werden, beispielsweise in Getreide, etwa Mais, Weizen, Roggen, Hafer in Reis, in Tomaten, Gurken, Bohnen Kartoffeln, Rüben im Wein- und Obstbau, in Rosen, Nelken und Chrysanthemen.

Die neuen Verbindungen zeigen Blattwirkung und systemische Wirkung. So wird mit zahlreichen erfindungsgemäßen Verbindungen bei der Blattbehandlung gegen Plasmopara mit einer Wirkstoffkonzentration zwischen 20 und 100 ppm eine vollständige Abtötung der Pilze erreicht. Bei der Bekämpfung von Phytophthora genügen im allgemeinen Wirkstoffkonzentrationen von 100 ppm, zum Teil weniger, für eine ausreichende Wirkung.

In manchen Fällen ist es günstig, die erfindungsgemäßen Verbindungen mit bekannten fungiziden Wirkstoffen zu kombinieren. Dabei geht die Wirkung der Kombinationen z.T. deutlich über die rein additive Wirkung hinaus.

Kombinationspartner

Manganethylenbisdithiocarbamat (Maneb)

Mangan-Zinkethylenbisdithiocarbamat (Mancozeb)

Zinkethylenbisdithiocarbamat (Zineb)

N-Trichlormethylthio-tetrahydrophthalimid (Captan)

N-Trichlormethylthiophthalimid (Folpet)

N-(1,1,2,2-Tetrachlorethylthio)tetrahydrophthalimid (Captafol)

2,3-Dicyano-1,4-dithiaanthrachinon (Dithianon)

Zink-(N,N'-propylen-bisdithiocarbamat (Propineb)

Kupferoxychlorid

Natrium-4-dimethylaminobenzoldiazoldiazosulfonat (Fenaminosulf)

Triphenylzinnactetat (Fentinacetat)

Triphenylzinnhydroxid (Fentinhydroxyd)

Eisendimethyldithiocarbamat (Ferbam)

N-(2-Furoyl)-N-(2,6-xylyl)-DL-alanin (Furalaxyl)

3-(Dimethylamino)propylcarbamat (Propamocarb)

N-Ethyl-N-(3-dimethylamino)thiocarbamat (Prothiocarb)

Tetramethylthiuramidsulfid (Thiram)

N-Dichlorfluormethylthio-N,N'-dimethyl-N-p-tolylsulfamid (Tolylfluamid)

N-(2-Methoxyacetyl)-N-(2,6-xylyl)alanin (Metalaxyl)

Zinkdimethylthiocarbamat (Ziram)

N-Dichlorfluormethylthio-N',N'-dimethyl-N-phenylsulfamid (Dichlorfluanid)

3-Trichlormethyl-5-ethoxy-1,2,4-thiadiazol (Etridazol)

Tri/aminzink-ethylenbis(dithiocarbamat)/tetrahydro-1,2,4,7-dithiadiazocin-3,8-dithion polymer (Metiram)

Aluminotris-(O-ethylphosphat) (Phosethyl)

2-Cyano-N-(ethylcarbamoyl)-2-methyloximino)-acetamid (Cymocanil)

N-(3-Chlorphenyl)-N-(tetrahydrofuran-2-on-3-yl)-cyclopropancarbonamid (Cyprofuran)

Tetrachlor-isophthalodinitril (Chlorothalonil)

6-Methyl-2-oxo-1,3-dithio[4,5-b]-chinoxalin (Chinomethionat)

4-Cyclododecyl-2,6-dimethylmorpholin (Dodemorph)

1-Dodecylguanidiniumacetat (Dodin)

Diisopropyl-5-nitroisophthalat (Nitrothal-isopropyl)

2,4-Dichlor-α-(pyrimidin-5-yl)benzhydrylalkohol (Fenarimol)

1-(ß-Allyloxy-2,4-dichlorphenethyl)imidazol (Imazalil)

3-(3,5-Dichlorpenyl)-N-isopropyl-2,4-dioxoimidazolidin-1-carboxamid (Iprodion)

Schwefel

2,3-Dihydro-6-methyl-5-phenylcarbamoyl-1,4-oxythiin-4,4-dioxid (Oxycarboxin)

N-(3,5-Dichlorphenyl)-1,2-dimethylcyclopropan-1,2-dicarboximid (Procymidon)

6-Ethoxycarbonyl-5-methylpyrazolo/1,5-/pyrimidin-2-yl-0,0-dimethylphosphorthioat (Pyrazophos)

2-(Thiazol-4-yl-benzimidazol (Thiabendazol)

1-(4-Chlorphenoxy-3,3-dimethyl-1-(1,2,4-triazol-1-yl-2-butanon (Triadimefon)

1-(4-Chlorphenoxy)-3,3-dimethyl-1-(1,2,4-triazol-1-yl-butanol (Triadimenol)

3-(3,5-Dichlorphenyl)-5-methyl-5-vinyloxyzolidin-2,4-dion (Vinclozolin)

Methylbenzimidazol-2-ylcarbamat (Carbendazin)

2,4,5-Trimethyl-N-phenyl-3-furancarboxamid (Methfuroxam)

ß-/1,1-Biphenyl/-4-yl-oxy)- -(1,1-dimethylethyl)-1H-1,2,4-triazol-1-ethanol (Bitertanol)

2-(2-Furyl)benzimidazol (Fuberidazol)

5-Butyl-2-ethylamino-6-methylpyrimidin-4-ol (Ethirimol)

2-Methyl-3-furanilid (Fenfuram)

Bis-(8-guanidino-octyl)amin (Guazatin)

N-Cyclohexyl-N-methoxy-2,5-dimethylfuran-3-carbonsäureamid (Furmecyclox)

2-Chlor-4'-fluor- -(pyrimidin-5-yl)benzhydrylalkohol (Nuarimol)

Methyl-1-(butylcarbamoyl)benzimidazolcarbamat (Benomyl)

O,O-Diethylphthalimidophosphonathioat (Dithalin)

7-Brom-5-chlorchinolin-8-yl-acrylat (Halacrimat)

1-/2-(2,4-Dichlorphenyl)4-propyl-1,3-dioxolan-2-yl-methyl/1H-1,2,4-triazol (Propiconazol)

Diemethyl-4,4'--(o-phenylen)bis(3-thioallophanat) (Thiophanat-methyl)

1,4-Bis(2,2,2-trichlor-1-formamidoethyl)piperzin (Triforine)

2,6-Dimethyl-4-tridecylmorpholin (Tridemorph)

4-/3-/4-(1,1-Dimethyl-ethyl)phenyl/-2-methyl/-propyl-2,6(cis-dimethyl-morpholin (Fenpropemorph)

1-/2-(2,4-Dichlorphenyl)-4-ethyl-1,3-dioxolan-2-ylmethyl/iH-1,2,4-trizol (Etaconazol)

1-/1-(2,4-Chlorphenyl)-4,4-dimethyl-3-hydroxy-2-pentyl/1,2,4-triazol (Diclobutrazol)

2,4-Dichlor-6-(2-chloranilino-1,3,5-triazin (Anilazin)

2-Jodo-N-phenylbenzamid (Benodanil)

2-sec.-butyl-4,6-dinitrophenyl-3-methylcrotonate (Binapacryl)

5-Butyl-2-(ethylamino)-6-methyl-4-pyrimidinyl dimethyl-sulfonat (Buprimat)

2,4-Dinitro-6-octylphenylcrotinat (Dinocap)

5,6-Dihydro-2-methyl-1,4-oxathiin-3-carbanilid (Carboxin)

N-Propyl-N-/(2,4,6-trichlorphenoxy)-2-ethyl/-imidazol-1-carbonamid (Prochloraz)

Für die Anwendung im Pflanzenschutz werden die neuen Verbindungen in üblicher Weise mit Hilfs- und/oder Trägerstoffen zu gebräuchlichen Formen von Schädlingsbekämpfungsmitteln verarbeitet, z.B. zu Lösungen, Emulsions- bzw. Lösungskonzentraten, Suspensionspulvern, Stäuben. Soweit Kombinationen mit anderen Wirkstoffen zur Anwendung gelangen sollen, kann dies in Form gemeinsamer Formulierungen oder z.B. in Form von Tankmischungen geschehen.

Die Konzentrate werden vor der Anwendung gegebenenfalls mit Wasser verdünnt, so daß Spritzbrühen mit einem Wirkstoffgehalt zwischen etwa 0,001 und 1 Gewichtsprozent erhalten werden. Bei der Anwendung als Low-volume- oder Ultra-Low-volume-Formulierung kann der Wirkstoffgehalt auch erheblich höher sein (bis ca. 20 bzw. bis ca. 90 Gewichtsprozent).

Beispiele für erfindungsgemäße Formulierungen:

1. Suspensionspulver

20 Gew.-Teile einer Verbindung der Formel I
20 Gew.-Teile Kaolin
5 Gew.-Teile Natriumsulfat
2 Gew.-Teile Schlämmkreide
9 Gew.-Teile Calciumligninsulfonat
1 Gew.-Teil Diisobutylnaphthalinnatrimsulfonat
43 Gew.-Teile Kieselkreide

Die Bestandteile werden vermahlen. Das Mittel wird für die Anwendung in so viel Wasser suspendiert, daß die Wirkstoffkonzentration etwa 0,001 bis 0,5 Gewichsprozent beträgt.

2. Emulsionskonzentrat

15 Gew.-Teile einer Verbindung der Formel I
10 Gew.-Teile Dodecylbenzolsulfonsäuretriethylaminsalz
75 Gew.-Teile Dimethylformamid

Die nachstehenden Beispiele sollen die erfindungsgemäßen Herstellungsverfahren näher erläutern.

Beispiel 1 (Verfahren a)

3-(3,4-Dimethoxyphenyl)-3-phenyl-acrylsäureN-(but-1-in-3-yl)-N-methylamid

Zu einer Lösung von 3-(3,4-Dimethoxyphenyl)-3-phenyl-acrylsäure (5,7g) und Triethylamin (3,5 ml) in THF[1)] (40 ml) wird unter Rühren und Kühlen bei etwa 5°C Innentemperatur Chlorkohlensäureethylester (2,1 ml in 5 ml THF) getropft. Nach 15 min. wird 1-Methylamino-1-methylprop-2-in (1,8 g in 5 ml THF) zugegeben, 15 min. bei Raumtemperatur nachgerührt und dann 1 h zum Sieden erhitzt. Nach Abdestillieren des Lösungsmittels wird mit Toluol/Wasser ausgeschüttelt und die eingeengte organische Phase mit Toluol/Aceton an Kieselgel gereinigt. Man erhält die Titelverbindung (3,5 g) als zähes Öl mit einem Rf = 0,65 (Kieselgel mit Toluol/Aceton = 7:3). Das Verhältnis der E/Z-Isomere wird 'H-NMR-spektrokopisch mit etwa 1:1 bestimmt.

1) THF = Tetrahydofuran

Beispiel 2 (Verfahren a)

3-(3,4-Dimethoxyphenyl)-3-phenylacrylsäure-4-chloranilid.

Ausgehend von 3-(3,4-Dimethoxyphenyl)-3-phenylacrylsäure (7,1 g) und 4-Chloranilin (3,2 g) erhält man analog Beispiel 1 die Titelverbindung (7,2 g) als langsam kristallisierendes Öl mit einem Rf = 0,70 (Kieselgel mit Toluol/Aceton = 7:3) und E/Z-Isomerenverhältnis = 1:1.

Beispiel 3 (Verfahren b)

3-(3,4-Chlorphenyl)-3-(3,4-dimethoxyphenyl)-2-methyl-acrylsäureimidazolid

Zu einer Lösung von 3-(4-Chlorphenyl)-3-(3,4-dimethoxyphenyl)-2-methyl-acrylsäure (8,3 g) in THF (40 ml) wird N,N'-Carbonyldiimidazol (4,85 in kleinen Protionen eingetragen. Nach Beendigung der $CO_2$-Entwicklung wird 15 min. zum Sieden erhitzt und dann im Vakuum eingedampft. Der Rückstand wird mit Toluol/Wasser ausgeschüttelt.
Aus der organischen Phase isoliert man die Titelverbindung (8,5 g) als zähes Öl mit einem Rf = 0,46 (Kieselgel mit Toluol/Aceton = 7:3) und einem E/Z-Isomerenverhältnis von etwa 1:1 ('H-NMR).

Analog zu den in den Beispielen 1 bis 3 beschriebenen Verfahrensvarianten a) b) und c) können die in nachstehender Tabelle angegebenen Verbindungen dargestellt werden, wobei Verfahren b) entweder zur Synthese der Imidazolide (z.B. Verb. No. 28, 29 oder 30) angewendet werden kann, oder aber die zunächst nach Verfahren b) erhältlichen Imidazolide in einer Folgereaktion mit dem gewünschten Amin der Formel (III) zu Produkten der Formel (I) weiter umgesetzt werden.

Tabelle I betrifft Verbindungen der Formel

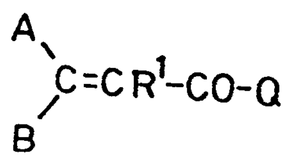

Diese Verbindungen fallen meist als Öl an. Zur Charakterisierung der Substanzen wird meistens der Rf-Wert angegeben, der in einem der folgenden Systemen bestimmt wird:

[1] Kieselgel mit Toluol/Aceton = 7/3

[2] Kieselgel mit Toluol/Aceton = 8/2

[3] Kieselgel mit Toluol/Aceton = 6/4

[4] Kieselgel mit Essigester/Isopropanol = 8/2

[5] Kieselgel mit Toluol/Aceton = 9/1

[6] Kiselgel mit Isopropylether

[7] Kiselgel mit Essigester/Isopropanol = 1/1

[8] Kieselgel mit Essigester

Tabelle 1

| Verb. No. | A | B | R | Q | physikalische Daten |
|---|---|---|---|---|---|
| 1 | $3,4-(CH_3O)_2-C_6H_3$ | $4-Cl-C_6H_4$ | H | $CH_3-N-CH_2CH_2-N(CH_3)_2$ | Öl |
| 2 | $3-Br,4-CH_3S-C_6H_3$ | $2,4-Cl_2-C_6H_3$ | H | $CH_3-N-CH_2CH_2-N(CH_3)_2$ | |
| 3 | $3-Br,4-NO_2-C_6H_3$ | $3-CH_3O-C_6H_4$ | H | $CH_3-N-CH_2CH_2-N(CH_3)_2$ | |
| 4 | $3-Br,4-(CH_3)_2N-C_6H_3$ | $4-CH_3OCH_2-C_6H_4$ | H | $CH_3-N-CH_2CH_2-N(CH_3)_2$ | |
| 5 | $3,4-(CH_3O)_2-C_6H_3$ | $4(4-Cl-C_6H_4O)-C_6H_4$ | $C_3H_7$ | $CH_3-N-CH_2CH_2-N(CH_3)_2$ | |
| 6 | $3,4-(C_2H_5)_2-C_6H_3$ | $4-CF_3-C_6H_4$ | H | $CH_3-N-CH_2CH_2-N(CH_3)_2$ | |
| 7 | $3,4-(CH_3O)_2-C_6H_3$ | $4-Cl-C_6H_4$ | H | $CH_3-N-CH_2CH_2-C_6H_5$ | Rf=0,60[3)] |
| 8 | $3,5-Cl_2,4-CH_3O-C_6H_3$ | $3,4-(CH_3)_2-C_6H_3$ | H | $CH_3-N-CH_2CH_2-C_6H_5$ | |
| 9 | $3-Cl,4-CH_3O-C_6H_3$ | $4-CO_2CH_3-C_6H_4$ | H | $CH_3-N-CH_2CH_2-C_6H_5$ | |
| 10 | $3-C_3H_7O,4-CH_3O-C_6H_3$ | $2-F-C_6H_4$ | H | $CH_3-N-CH_2CH_2-C_6H_5$ | |
| 11 | $3-Br,4-CH_3O-C_6H_3$ | $3-CH_3-C_6H_4$ | H | $CH_3-N-CH_2CH_2-C_6H_5$ | |
| 12 | $3-NH_2,4-CH_3O-C_6H_3$ | $3-Cl-C_6H_4$ | H | $CH_3-N-CH_2CH_2-C_6H_5$ | |
| 13 | $3,4-(CH_3O)_2-C_6H_3$ | $4-Cl-C_6H_4$ | H | $NH-CH_2CH_2-N(C_2H_5)_2$ | Öl |
| 14 | $3,4-(CH_3O)_2-C_6H_3$ | $3,4-Cl-C_6H_3$ | $CH_3$ | $NH-CH_2CH_2-N(C_2H_5)_2$ | |
| 15 | $3-Cl,4-CH_3O-C_6H_3$ | $4-I-C_6H_4$ | H | $NH-CH_2CH_2-N(C_2H_5)_2$ | |

| Verb. No. | A | B | R | Q | physikalische Daten |
|---|---|---|---|---|---|
| 16 | 3-$C_2H_5$,4-$CH_3OC_6H_3$ | 4-$C_2H_5S$-$C_6H_4$ | H | NH-$CH_2CH_2$-N$(C_2H_5)_2$ | |
| 17 | 3,4-$(CH_3O)_2$-$C_6H_3$ | 4-Cl-$C_6H_4$ | H | NH-$CH_2CH_2CH_2$-N$(CH_3)_2$ | Fp:133-136°C |
| 18 | 3,4-$(CH_3O)_2C_6H_3$ | 3-Dibenzofuryl | H | NH-$CH_2CH_2CH_2$-N$(CH_3)_2$ | |
| 19 | 2,5-$(CH_3O)_2$-$C_6H_3$ | 4-$CH_3SO$-$C_6H_4$ | H | NH-$CH_2CH_2CH_2$-N$(CH_3)_2$ | |
| 20 | 3,5-$Cl_2$-$C_6H_3$ | 4-$C_3H_7O$ -$C_6H_5$ | H | NH-$CH_2CH_2CH_2$-N$(CH_3)_2$ | |
| 21 | 3,4-$(CH_3O)_2$-$C_6H_3$ | $C_6H_5$ | H | NH-(4-Cl-$C_6H_4$) | Rf=0,70 1) |
| 22 | 3,4-$Cl_2$-$C_6H_3$ | 3-Cl-$C_6H_4$ | H | NH-(4-Cl-$C_6H_4$) | |
| 23 | 3-$C_2H_5$,4-$CH_3O$-$C_6H_3$ | 4-$C_6H_5SO_2$-$C_6H_4$ | H | NH-(4-Cl-$C_6H_4$) | |
| 24 | 3,4-$(CH_3O)_2$-$C_6H_3$ | $C_6H_5$ | H | NH-(3-Cl-$C_6H_4$) | Rf=0,76 1) |
| 25 | 3,4-$(CH_3O)_2$-$C_6H_3$ | 3,4-$(CH_3O)_2$-$C_6H_3$ | H | NH-(3-Cl-$C_6H_4$) | |
| 26 | 4-$NO_2$-$C_6H_4$ | 3,5-$Cl_2$,4-OH -$C_6H_2$ | H | NH-(3-Cl-$C_6H_4$) | |
| 27 | 3,4-$(CH_3O)_2$-$C_6H_3$ | $C_6H_5$ | H | NH-(2-Cl-$C_6H_4$) | Rf=0,81 1) |
| 28 | 3-$CH_3$-4-$CH_3O$-$C_6H_3$ | $C_6H_5$ | H | 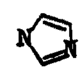 | F=120-123°C |
| 29 | 4-Cl-$C_6H_4$ | 4-Cl-$C_6H_4$ | $CH_3$ | 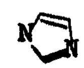 | |
| 30 | 4-Cl-$C_6H_4$ | 2-Cl-$C_6H_4$ | $C_2H_5$ | 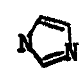 | |

| Verb. No. | A | B | R | Q | physikalische Daten |
|---|---|---|---|---|---|
| 31 | $3,4-(CH_3O)_2-C_6H_3$ | $C_6H_5$ | H | $CH_3NCH_2CH_2-O-CH_3$ | Rf=0,29 1) |
| 32 | $3,5-Cl,4-NH_2-C_6H_2$ | $4-C_6H_5-C_6H_4$ | H | $CH_3NCH_2CH_2-O-CH_3$ | |
| 33 | $3,4,5-(CH_3O)_3-C_6H_2$ | $4-C_2H_5-C_6H_4$ | H | $CH_3NCH_2CH_2-O-CH_3$ | |
| 34 | $4-(CH_3)_2N-C_6H_4$ | $4-(CH_3)_2CH-C_6H_4$ | H | $CH_3NCH_2CH_2-O-CH_3$ | |
| 35 | $3-CH_3-4-CH_3O-C_6H_3$ | $C_6H_5$ | H | 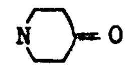 | Fp: 150-154°C |
| 36 | $3-NH_2,4-CH_3O-C_6H_3$ | $4-Cyclohexyl-C_6H_4$ | H | 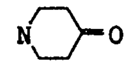 | |
| 37 | $3-Cl,4-CH_3O-C_6H_3$ | $4-C_6H_5-S-C_6H_4$ | H | 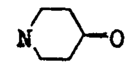 | |
| 38 | $3-CH_3O,4-CH_3-C_6H_3$ | 2-Benzofuryl | H | 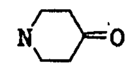 | |
| 39 | $3-CH_3-4-CH_3O-C_6H_3$ | $C_6H_5$ | H | 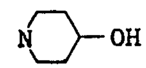 | Fp: 64°C |
| 40 | $3-C_2H_5O,4-CH_3O-C_6H_3$ | $4-CH_3SO_2-C_6H_4$ | H | 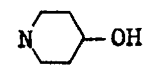 | |
| 41 | $3,4-(CH_3O)_2-C_6H_3$ | 2-Fluorenyl | H | 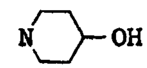 | |
| 42 | $3-Br,4-NH_2-C_6H_3$ | $3-CH_3O-C_6H_4$ | H | 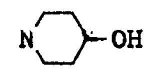 | |
| 43 | $3,5-Br_2,4-NH_2-C_6H_2$ | $4-(CH_3)_2CHOC_6H_4$ | H | 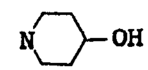 | Rf=0,28 1) |

| Verb. No. | A | B | R | Q | physikalische Daten |
|---|---|---|---|---|---|
| 45 | 3-CN, 4-$CH_3$-$C_6H_3$ | 3-F-$C_6H_4$ | H | 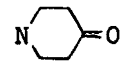 | |
| 46 | 3-$CH_3$, 4-$C_2H_5O$-$C_6H_3$ | 3-Cl, 4-F-$C_6H_3$ | | 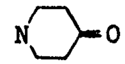 | |
| 47 | 3,4-$(CH_3O)_2$-$C_6H_3$ | $C_6H_5$ | H | NH-$CH_2CH_2$-O-$CH_3$ | Rf=0,3 1) |
| 48 | 3-Br, 4-$C_2H_5$-O-$C_6H_3$ | 4-$(CH_3)_2N$-$C_6H_4$ | | NH-$CH_2CH_2$-O-$CH_3$ | |
| 49 | 3-$CH_3$-4-$CH_3O$-$C_6H_3$ | $C_6H_5$ | H | NH-$CH_2CH_2$-O-$CH_3$ | Rf=0,33 1) |
| 50 | 3-$CH_3$, 4-$CH_3CO_2$-$C_6H_3$ | 4-$C_2H_5$-$C_6H_4$ | | NH-$CH_2CH_2$-O-$CH_3$ | |
| 51 | 3,4-$(CH_3O)_2$-$C_6H_3$ | $C_6H_5$ | H | NH-$(CH_2)_3$-OH | Rf=0,59 4) |
| 52 | 3-$CH_3$, 4-$CF_2HO$-$C_6H_3$ | 4-$(CH_3)_3$-$C_6H_4$ | H | NH-$(CH_2)_3$-OH | |
| 53 | 3-Br, 4-$CH_3O$-$C_6H_3$ | 4-$CF_3O$-$C_6H_4$ | $CH_3$ | NH-$(CH_2)_3$-OH | |
| 54 | 3-$C_2H_5O$, 4-$CH_3O$-$C_6H_3$ | 3-$NO_2$-$C_6H_4$ | H | NH-$(CH_2)_3$-OH | |
| 55 | 3,4-$(CH_3O)_2$-$C_6H_3$ | $C_6H_5$ | H | 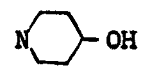 | Rf=0,1 1) |
| 56 | 3-Br, 4-$CH_3O$-$C_6H_3$ | 2-Furyl | H | 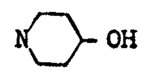 | |
| 57 | 3-Cl, 4-$NH_2$-$C_6H_3$ | 3-Br-$C_6H_4$ | H | 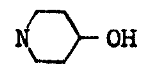 | |
| 58 | 3-$CH_3$, 4-$CH_3O$-$C_6H_3$ | 4-$C_6H_5$-O-$C_6H_4$ | H | 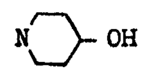 | |

| Verb. No. | A | B | R | Q | physikalische Daten |
|---|---|---|---|---|---|
| 59 | $3-CH_3-4-CH_3O-C_6H_3$ | $C_6H_5$ | H | 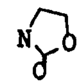 | Fp: 95-110°C |
| 60 | $3,4-(CH_3)_2-C_6H_3$ | 2-Thienyl | H | 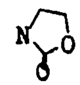 | |
| 61 | $3,5-Cl_2-C_6H_3$ | $4-CH_3O-C_6H_4$ | H | 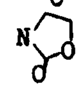 | |
| 62 | $3-CH_2-4-CH_3O-C_6H_3$ | $C_6H_5$ | H | $NHCH_2COOC_2H_5$ | Rf= 0,58 5) |
| 63 | $3-Cl,4-CH_3O-C_6H_3$ | $3,4-Cl_2-C_6H_3$ | H | $NHCH_2COOC_2H_5$ | |
| 64 | $3-Br,4-(CH_3)_2N-C_6H_3$ | $3-CH_3-C_6H_4$ | H | $NHCH_2COOC_2H_5$ | |
| 65 | $3,5-(CH_3)_2,4-CH_3O-C_6H_3$ | $4-NO_2-C_6H_4$ | H | $NHCH_2COOC_2H_5$ | |
| 66 | $3-CH_3-4-CH_3O-C_6H_3$ | $C_6H_5$ | H | $NH-C_{12}H_{25}$ | Rf=0,42 6) |
| 67 | $3,5-Cl_2,4-NH_2-C_6H_2$ | $C_6H_5$ | H | $NH-C_{12}H_{25}$ | |
| 68 | $4-CH_3O-C_6H_4$ | $2-Cl-C_6H_4$ | $CH_3$ | $NH-C_{12}H_{25}$ | |
| 69 | $3-CH_3-4-CH_3O-C_6H_3$ | $C_6H_5$ | H | $CH_3-N-OCH_3$ | Rf=0,31 7) |
| 70 | $3,5-(CH_3)_2-C_6H_3$ | $3-NO_2-C_6H_4$ | H | $CH_3-N-OCH_3$ | |
| 71 | $3-NO_2,4-CH_3OC_6H_3$ | $3-CF_3-C_6H_4$ | H | $CH_3-N-OCH_3$ | |

| Verb. No. | A | B | R | Q | physikalische Daten |
|---|---|---|---|---|---|
| 72 | $3-CH_3-4-CH_3O-C_6H_3$ | $C_6H_5$ | H | $N(C_6H_5)(3,4-(CH_3O)_2C_6H_3$ | Fp.: 227°C (Zers., |
| 73 | $3-F,4-CH_3O-C_6H_3$ | $4-C_4H_9-C_6H_4$ | H | $N(C_6H_5)(3,4-(CH_3O)_2C_6H_3$ | |
| 74 | $3-CH_3O,4-C_2H_5OC_6H_3$ | $4-C_4H_3O-C_6H_4$ | H | $N(C_6H_5)(3,4-(CH_3O)_2C_6H_3$ | |
| 75 | $3-J,4-CH_3O-C_6H_3$ | $3-CH_3S-C_6H_4$ | H | $N(C_6H_5)(3,4-(CH_3O)_2C_6H_3$ | |
| 76 | $3,4-(CH_3O)_2-C_6H_3$ | $4-Cl-C_6H_4$ | H | $N(C_6H_5)_2$ | |
| 77 | $3-CH_3,4-NH_2-C_6H_3$ | $4-F-C_6H_4$ | H | $N(C_6H_5)_2$ | |
| 78 | $2,4-(CH_3O)_2-C_6H_3$ | $3-Cl-C_6H_4$ | H | $N(C_6H_5)_2$ | |
| 79 | $3-CH_3,4-NO_2-C_6H_3$ | $4-CF_2HO-C_6H_4$ | H | $N(C_6H_5)_2$ | |
| 80 | $3-C_2H_5-,4-CH_3O-C_6H_3$ | 2-Naphthyl | H | $N(C_6H_5)_2$ | |
| 81 | $3-C_3H_7(n),4-CH_3O-C_6H_3$ | 2-Benzothienyl | H | $N(C_6H_5)_2$ | |
| 82 | $3,4-(CH_3O)_2C_6H_3$ | $4-Br-C_6H_4$ | H | $CH_3NCH_2-$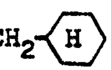 | |
| 83 | $3,4-(CH_3O)_2-C_6H_3$ | $4-Cl-C_6H_4$ | H | $NH-C(CH_3)_2-C\equiv CH$ | FP.: 157°C |
| 84 | $3,4-(CH_3O)_2C_6H_3$ | $4-C_6H_5-O-C_6H_4$ | H | $NH-C(CH_3)_2-C\equiv CH$ | |
| 85 | $3-CH_3,4-CH_3O-C_6H_3$ | $3,4-Cl_2-C_6H_3$ | H | $NH-C(CH_3)_2-C\equiv CH$ | |
| 86 | $3-Br,4-CH_3O-C_6H_3$ | $3-NO_2-C_6H_4$ | H | $NH-C(CH_3)_2-C\equiv CH$ | |
| 87 | $3-Cl,4-CH_3O-C_6H_3$ | $4-NO_2-C_6H_4$ | H | $NH-C(CH_3)_2-C\equiv CH$ | |

| Verb. No. | A | B | R | Q | physikalische Daten |
|---|---|---|---|---|---|
| 88 | $3,4-(CH_3O)_2-C_6H_3$ | $4-Cl-C_6H_4$ | H | $CH_3-N-CH_2-C\equiv CH$ | Rf=0,51 3) |
| 89 | $3,4-(CH_3O)_2C_6H_3$ | $4-C_6H_5-C_6H_4$ | H | $CH_3-N-CH_2-C\equiv CH$ | |
| 90 | $3-CH_3O,4-CH_3-C_6H_3$ | $4-Cl-C_6H_4$ | H | $CH_3-N-CH_2-C\equiv CH$ | |
| 91 | $3-Br,4-CH_2O-C_6H_3$ | $4-C_4H_9C_6H_4$ | H | $CH_3-N-CH_2-C\equiv CH$ | |
| 92 | $3-CH_3,4-C_2H_5O-C_6H_3$ | $3-CH_3-C_6H_4$ | H | $CH_3-N-CH_2-C\equiv CH$ | |
| 93 | $3,4-(CH_3O)_2-C_6H_3$ | $4-Cl-C_6H_4$ | $C_2H_5$ | 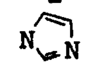 | Rf=0,50 1) |
| 94 | $4-Cl-C_6H_4$ | $2-Cl-C_6H_4$ | $C_3H_7$ | 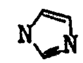 | |
| 95 | $4-F-C_6H_4$ | $2,4-Cl_2C_6H_3$ | $CH(CH_3)_2$ | 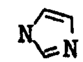 | |
| 96 | $2-F-C_6H_4$ | $4-F-C_6H_4$ | $C(CH_3)_3$ | 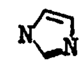 | |
| 97 | $3,4-Cl_2-C_6H_3$ | $Br-C_6H_4$ | $C_4H_9$ | 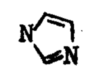 | |
| 98 | $3,4-(CH_3O)_2-C_6H_3$ | $4-Cl-C_6H_4$ | H | $CH_3NCH_2$ 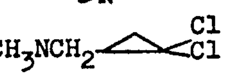 | Rf=0,31 2) |
| 99 | $3,4-(CH_3O)_2C_6H_3$ | $4-C_6H_5-C_6H_4$ | H | $CH_3NCH_2$ 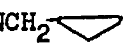 | |
| 100 | $3,4-(CH_3O)_2C_6H_3$ | $4-CH_3-C_6H_4$ | H | $CH_3NCH_2$ 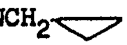 | |
| 101 | $3-Br,4-CH_3O-C_6H_3$ | $4-C_2H_5O_2C-C_6H_4$ | H | $CH_3NCH_2$ 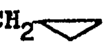 | |
| 102 | $3-Br,4-(CH_3)_2N\ C_6H_3$ | $3-Cl-C_6H_4$ | H | $CH_3NCH_2$ 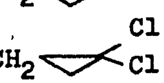 | |

| Verb. No. | A | B | R | Q | physikalische Daten |
|---|---|---|---|---|---|
| 103 | $3,4-(CH_3O)_2-C_6H_3$ | $4-Cl-C_6H_4$ | H | 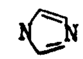 | Isomer 1 F=184-186°C |
| 104 | $3,4(CH_3O)_2-C_6H_3$ | $4-Cl-C_6H_4$ | H | 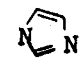 | Isomer 2 F=134°C |
| 105 | $4-CH_3-C_6H_4$ | $2,4-Cl_2-C_6H_2$ | $CH(CH_3)_2$ | 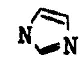 | |
| 106 | $4-C_2H_5O-C_6H_4$ | $4-Cl-C_6H_4$ | $C_3H_7$ | 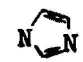 | |
| 107 | $2,4-(CH_3),-C_6H_4$ | $4-C_6H_5-C_6H_4$ | $C_2H_5$ | 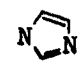 | |
| 108 | $2,4-Cl_2-C_6H_3$ | $4-Br-C_6H_4$ | $C_3H_7$ | 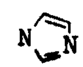 | |
| 109 | $3,4-(CH_3O)_2C_6H_3$ | 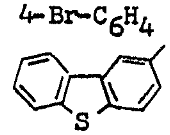 | H | $CH_3NCHCH_3-C{\equiv}CH$ | |
| 110 | $3,4-(CH_3O)_2-C_6H_3$ | $C_6H_5$ | H | $CH_3-N-CHCH_3C{\equiv}CH$ | |
| 111 | $3,4-(CH_3O)_2C_6H_3$ | 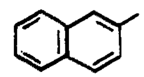 | H | $CH_3-N-CHCH_3C{\equiv}CH$ | |
| 112 | $3,5-Cl_2,4-NH_2-C_6H_2$ | $4-Cl-C_6H_4$ | H | $CH_3-N-CHCH_3C{\equiv}CH$ | |
| 113 | $3-C_2H_5,4-CH_3O-C_6H_3$ | $3-Br-C_6H_4$ | H | $CH_3-N-CHCH_3C{\equiv}CH$ | |
| 114 | $3,4-(CH_3O)_2-C_6H_3$ | $3-CF_3C_6H_4$ | $CH_3$ | $CH_3-N-CHCH_3C{\equiv}CH$ | |
| 115 | $3,5-(CH_3)_2,4-CH_3O-C_6H_2$ | $4-C_5H_{11}O-C_6H_4$ | H | $CH_3-N-CHCH_3C{\equiv}CH$ | |

| Verb. No. | A | B | R | Q | physikalisc he Daten |
|---|---|---|---|---|---|
| 116 | $3,4-(CH_3O)_2-C_6H_3$ | $4-Cl-C_6H_4$ | H | $CH_3-N-CH_2-CH$ 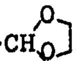 | Rf=0,5 3) |
| 117 | $3-CH_3,4-CH_3O-C_6H_3$ | $4-Br-C_6H_4$ | H | $CH_3-N-CH_2-CH$ 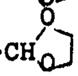 | |
| 118 | $3-Br,4-CH_3O-C_6H_3$ | $3,4-Cl_2C_6H_3$ | H | $CH_3-N-CH_2-CH$ 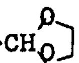 | |
| 119 | $3-Cl,4-CH_3O-C_6H_3$ | $4-F-C_6H_4$ | H | $CH_3-N-CH_2-CH$ 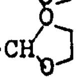 | |
| 120 | $3-C_2H_5,4-CH_3OC_6H_3$ | $4-C_3H_7-C_6H_4$ | H | $CH_3-N-CH_2-CH$ 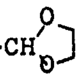 | |
| 121 | $3-CH_3O,4-CH_3-C_6H_3$ | $4-(CH_3)_2N-C_6H_4$ | H | $CH_3-N-CH_2-CH$ 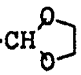 | |
| 122 | $3,5-Cl,4-NH_2-C_6H_2$ | $C_6H_5$ | H | $CH_3-N-CH_2-CH$ 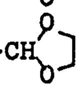 | |
| 123 | $3,4-(CH_3O)_2-C_6H_3$ | $4-Cl-C_6H_4$ | H | $CH_3-N-CH_2CN$ | Rf=0,35 3) |
| 124 | $3,4-(CH_3O)_2-C_6H_3$ | $-CH=CH-4-Cl-C_6H_4$ | H | $CH_3-N-CH_2CN$ | |
| 125 | $3,4-(CH_3O)_2-C_6H_3$ | $-CH=CH-C_6H_5$ | H | $CH_3-N-CH_2CN$ | |
| 126 | $3-Br,4-CH_3O-C_6H_3$ | $4-(CH_3)_3C-C_6H_4$ | H | $CH_3-N-CH_2CN$ | |
| 127 | $3-Cl,4-CH_3O-C_6H_3$ | $4-CH_3-C_6H_4$ | H | $CH_3-N-CH_2CN$ | |

| Verb. No. | A | B | R | Q | physikalische Daten |
|---|---|---|---|---|---|
| 128 | $3,4-(CH_3O)_2-C_6H_3$ | $4-Cl-C_6H_4$ | H | 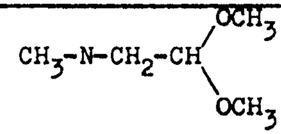 | Rf=0,51 3) |
| 129 | $3-Br,4-NH_2-C_6H_3$ | $4-C_3H_7O-C_6H_4$ | H | 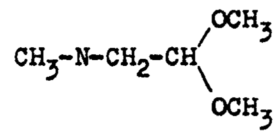 | |
| 130 | $3,4-(CH_3O)-C_6H_3$ | 2-Furyl | H | 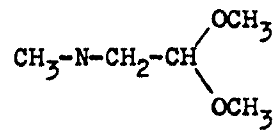 | |
| 131 | $3-C_3H_7,4-CH_3O-C_6H_3$ | 2-Thienyl | H | 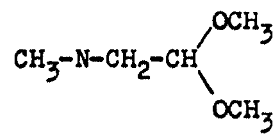 | |
| 132 | $3,5-(CH_3)_2-C_6H_3$ | $4-NO_2-C_6H_4$ | H | 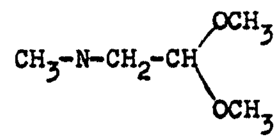 | |
| 133 | $3,4-(CH_3O)_2-C_6H_3$ | $4-Cl-C_6H_4$ | H | 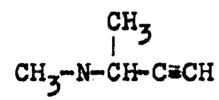 | |
| 134 | $3,4-(CH_3O)_2-C_6H_3$ | $-CH=CH-4-Cl-C_6H_4$ | H | 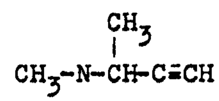 | |

| Verb. No. | A | B | R | Q | Physikalische Daten |
|---|---|---|---|---|---|
| 135 | $3,4-(CH_3O)_2C_6H_3$ | $4-C_6H_5-C_6H_4$ | H | $CH_3$<br>$CH_3-N-CH-C\equiv CH$ | |
| 136 | $3-CH_3,4-CH_3O-C_6H_3$ | $4-CF_3O-C_6H_4$ | H | $CH_3$<br>$CH_3-N-CH-C\equiv CH$ | |
| 137 | $3,4-(CH_3O)_2-C_6H_3$ | $4-Cl-C_6H_4$ | H | $CH_3$<br>$CH_3-N-CH-C\equiv CH$ | |
| 138 | $3,4-(CH_3O),C_6H_3$ | $4-Br-C_6H_4$ | $CH_3$ | $CH_3$<br>$CH_3-N-CH-C\equiv CH$ | Rf=0,51 1) |
| 139 | $3-C_2H_5O,4-CH_3OC_6H_3$ | $4-CH_3C_6H_4$ | H | $CH_3$<br>$CH_3-N-CH-C\equiv CH$ | |
| 140 | $4-Cl-C_6H_4$ | $3,5-Cl_2,4-NH_2-C_6H_2$ | H | $CH_3$<br>$CH_3-N-CH-C\equiv CH$ | |
| 141 | $3,4-(CH_3O)_2-C_6H_3$ | $4-Cl-C_6H_4$ | H | $NH-CH_2-COOC_2H_5$ | Öl |
| 142 | $3,4-(CH_3O)_2C_6H_3$ | $4-C_6H_5-C_6H_4$ | H | $NH-CH_2-COOC_2H_5$ | |
| 143 | $3,4-(CH_3O)_2C_6H_3$ | $4-C_6H_3-O-C_6H_4$ | H | $NH-CH_2-COOC_2H_5$ | |
| 144 | $3,4-(CH_3O)_2C_6H_3$ | $4-C_4H_9-C_6H_4$ | H | $NH-CH_2-COOC_2H_5$ | |

| Verb. No. | A | B | R | Q | physikalische Daten |
|---|---|---|---|---|---|
| 145 | 3-Br, 4-$CH_3OC_6H_3$ | 4-$CH_3S$-$C_6H_4$ | H | NH-$CH_2$-$COOC_2H_5$ | |
| 146 | 3-Cl, 4-$CH_3OC_6H_3$ | $C_6H_5$ | H | NH-$CH_2$-$COOC_2H_5$ | |
| 147 | 3,4-$(CH_3O)_2$-$C_6H_3$ | 4-Cl-$C_6H_4$ | H | NH-$CH_2$-CO-N 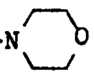 | Fp=58-65°C |
| 148 | 3,4-$(CH_3O)_2C_6H_3$ | 4-(4-$ClC_6H_4S$)-$C_6H_4$ | H | NH-$CH_2$-CO-N 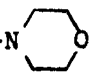 | |
| 149 | 3-$CH_3O$, 4-$CH_3$-$C_6H_3$ | 4-Br-$C_6H_4$ | H | NH-$CH_2$-CO-N 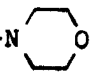 | |
| 150 | 3-Br, 4-$CH_3O$-$C_6H_3$ | 4-$CH_3$-$C_6H_4$ | H | NH-$CH_2$-CO-N 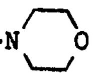 | |
| 151 | 3-Cl, 4-$C_2H_5$-$OC_6H_3$ | 3-$CH_3$-$C_6H_4$ | H | NH-$CH_2$-CO-N 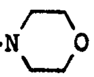 | |
| 152 | 3,4-$(CH_3O)_2$-$C_6H_3$ | 4-Cl-$C_6H_4$ | H | NH-$CH_2$-COOH | Fp=72-90°C |
| 153 | 3,4-$(CH_3O)_2C_6H_3$ | 4-$CH_3O$-$C_6H_4$ | H | NH-$CH_2$-COOH | |
| 154 | 3-$CH_3$, 4-$CH_3OC_6H_3$ | $C_6H_5$ | $C_2H_5$ | NH-$CH_2$-COOH | |
| 155 | 3-Cl, 4-$CH_3OC_6H_3$ | 4-Cl-$C_6H_4$ | $CH_3$ | NH-$CH_2$-COOH | |
| 156 | 3-Cl, 4-$C_2H_5OC_6H_3$ | 4-$CH_3OCH_2$-$C_6H_4$ | n-$C_3H_7$ | NH-$CH_2$-COOH | |
| 157 | 3,4-$(CH_3O)_2$-$C_6H_3$ | 4-Cl-$C_6H_4$ | H | $CH_3$-N-$CH_2$-$CH_2$-CN | Rf=0,25 2) |

| Verb. No. | A | B | R | Q | physikalische Daten |
|---|---|---|---|---|---|
| 158 | 3,4-$(CH_3O)_2C_6H_3$ | 4-$CH_3O-C_6H_4$ | H | $CH_3-N-CH_2-CH_2-CN$ | |
| 159 | 3,4-$(CH_3O)_2C_6H_3$ | 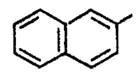 | H | $CH_3-N-CH_2-CH_2-CN$ | |
| 160 | 3,4-$(CH_3)_2C_6H_3$ | 4-$CO_2CH_3-C_6H_4$ | H | $CH_3-N-CH_2-CH_2-CN$ | |
| 161 | 3,4-$(CH_3O)_2-C_6H_3$ | 3,4-$Cl_2-C_6H_4$ | H | $CH_3-N-CH_2-CH_2-CN$ | |
| 162 | 3,4-$(CH_3O)_2-C_6H_3$ | $-CH=CH-C_6H_5$ | H | $CH_3-N-CH_2-CH_2-CN$ | |
| 163 | 3-Br,4-$(CH_3)_2N-C_6H_3$ | 4-$C_2H_5-C_6H_4$ | H | $Cn_3-N-CH_2-CH_2-CN$ | |
| 164 | 3,4-$(CH_3O)_2-C_6H_3$ | 4-$Cl-C_6H_4$ | H | $CH_3-N-CH_2-CO-N$ 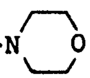 | |
| 165 | 3,4-$(CH_3O)_2C_6H_3$ | 4-$C_6H_3-C_6H_4$ | H | $CH_3-N-CH_2-CO-N$ 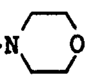 | |
| 166 | 3,4-$(CH_3O)_2-C_6H_3$ | 4-$(CH_3)_3C-C_6H_4$ | H | $CH_3-N-CH_2-CO-N$ 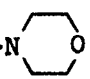 | |
| 167 | 3,4-$(CH_3O)_2-C_6H_3$ | 4-$C_6H_5SO_2C_6H_4$ | H | $CH_3-N-CH_2-CO-N$ 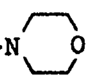 | |
| 168 | 3,4-$(CH_3O)_2-C_6H_3$ | 4-$Cl-C_6H_4$ | H | $CH_3-N-CH_2-CO-N(CH_3)C_2H_5$ | |

| Verb. No. | A | B | R | Q | physikalische Daten |
|---|---|---|---|---|---|
| 169 | $3-CH_3,4-CH_3O-C_6H_3$ | $4-Br-C_6H_4$ | H | $CH_3-N-CH_2-CO-N(CH_3)C_2H_5$ | |
| 170 | $3-Br,4-CH_3O-C_6H_3$ | $4-(CH_3),CH-C_6H_4$ | H | $CH_3-N-CH_2-CO-N(CH_3)C_2H_5$ | |
| 171 | $3-Cl,4-CH_3OC_6H_3$ | 4-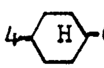$-C_6H_4$ | H | $CH_3-N-CH_2-CO-N(CH_3)C_2H_5$ | |
| 172 | $4-Br-C_6H_4$ | $3,5-Cl_2,4-NH_2-C_6H_2$ | H | $CH_3-N-CH_2-CO-N(CH_3)C_2H_5$ | |
| 173 | $3,4-(CH_3O)_2-C_6H_3$ | $4-Cl-C_6H_4$ | H | $CH_3-N-CH_2-\overset{O}{\overset{\|}{C}}-\overset{CH_3}{\overset{\mid}{N}}-CH(CH_3)_2$ | |
| 174 | $3,4-(CH_3O)_2C_6H_3$ | $3,4-Cl_2-C_6H_4$ | H | $CH_3-N-CH_2-\overset{O}{\overset{\|}{C}}-\overset{CH_3}{\overset{\mid}{N}}-CH(CH_3)_2$ | |
| 175 | $3,4-(CH_3O)_2C_6H_3$ | $C_6H_5$ | H | $CH_3-N-CH_2-\overset{O}{\overset{\|}{C}}-\overset{CH_3}{\overset{\mid}{N}}-CH(CH_3)_2$ | |
| 176 | $3,4-(CH_3)_2-C_6H_3$ | $3,4-OCH_2O-C_6H_4$ | H | $CH_3-N-CH_2-\overset{O}{\overset{\|}{C}}-\overset{CH_3}{\overset{\mid}{N}}-CH(CH_3)_2$ | |
| 177 | $3,4-(CH_3O)_2-C_6H_3$ | $4-Cl-C_6H_4$ | H | $NH-CH_2-CO-NH-CH(CH_3)_2$ | Fp=95-111°C |
| 178 | $3,4-(CH_3O)_2C_6H_3$ | $4-(CH_3)_2NSO_2-C_6H_4$ | H | $NH-CH_2-CO-NH-CH(CH_3)_2$ | |

| Verb. No. | A | B | R | Q | physikalische Daten |
|---|---|---|---|---|---|
| 179 | $3,4-(CH_3O)_2C_6H_3$ | $3-CH_3-C_6H_4$ | H | $NH-CH_2-CO-NH-CH(CH_3)_2$ | |
| 180 | $3-CH_3,4-CH_3OC_6H_3$ | $4-F-C_6H_4$ | H | $NH-CH_2-CO-NH-CH(CH_3)_2$ | |
| 181 | $3,4-(CH_3O)_2-C_6H_3$ | $4-Cl-C_6H_4$ | H | $NH-CH_2-CO-NHC_3H_7$ | Fp=76-89°C |
| 182 | $3,4-(CH_3O)_2C_6H_3$ | $4-CNC_6H_4$ | H | $NH-CH_2-CO-NHC_3H_7$ | |
| 183 | $3,4-(CH_3O)_2C_6H_3$ | $4-CH_2=CHCH_2O-C_6H_4$ | H | $NH-CH_2-CO-HCH_3H_7$ | |
| 184 | $3,4-(CH_3O)_2C_6H_3$ | $4-CON(CH_3)_2-C_6H_4$ | H | $NH-CH_2-CO-H\,CH_3H_7$ | |
| 185 | $3,4-(CH_3)_2-C_6H_3$ | $4-Cl-C_6H_4$ | H | $NH-CH_2-CO-N$ 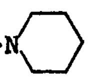 | Rf=0,30 [8)] |
| 186 | $3,4-(CH_3O)_2C_6H_3$ | $4-C_6H_5O-C_6H_4$ | H | $NH-CH_2-CO-N$ 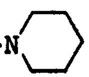 | |
| 187 | $3-Br,4-CH_3OC_6H_3$ | $4-(CH_3)_2CH-C_6H_4$ | H | $NH-CH_2-CO-N$ 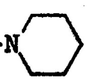 | |
| 188 | $3,4-(CH_3O)_2-C_6H_3$ | $4-Cl-C_6H_4$ | H | $NH-CH_2-CO-N(C_2H_5)_2$ | Rf=0,28 [8)] |
| 189 | $3-CH_3,4-CH_3OC_6H_3$ | $3,4-Cl_2-C_6H_3$ | H | $NH-CH_2-CO-N(C_2H_5)_2$ | |
| 190 | $3,4-(CH_3O)_2C_6H_3$ | $3,4-(CH_2)_2-C_6H_3$ | H | $NH-CH_2-CO-N(C_2H_5)_2$ | |
| 191 | $3,4-(CH_3O)_2C_6H_3$ | $3,4-(CH_2)_4-C_6H_3$ | H | $CH_3NCH_2CONCH_3$ | |

| Verb. No. | A | B | $R^1$ | Q | physikalische Daten |
|---|---|---|---|---|---|
| 192 | $3,4-(CH_3O)_2C_6H_3$ | $4-CF_3-C_6H_4$ | H | $CH_3NCH_2CONHCH_3$ | |
| 193 | $3,4-(CH_3O)_2-C_6H_5$ | $4-Cl-C_6H_4$ | H | $NH-CH_2-CO-NHCH_3$ | Fp = 134-148°C |
| 194 | $3,4-(CH_3O)_2C_6H_3$ | $4-(4-C_2H_5C_6H_4)C_6H_4$ | H | $NH-CH_2-CO-NHCH_3$ | |
| 195 | $3,4-(CH_3O)_2C_6H_3$ | $4-Cl-C_6H_4$ | H | 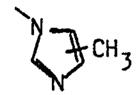 | Fp = 182 |
| 196 | $3,4-(CH_3O)_2C_6H_3$ | $4-Cl-C_6H_4$ | H | $N(CH_3)CH_2CH_2OH$ | |
| 197 | $3,4-(CH_3O)_2-C_6H_3$ | $4-Cl-C_6H_4$ | H | $NHCH_2-COCH_3$ | |
| 198 | $3,4-(CH_3O)_2-C_6H_3$ | $4-Cl-C_6H_4$ | H | $N(CH_3)CH_2-CO-CH_3$ | |
| 199 | $3,4-(CH_3O)_2-C_6H_3$ | $4-Cl-C_6H_4$ | H | 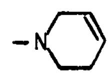 | |
| 200 | $3,4-(CH_3O)_2-C_6H_3$ | $4-Cl-C_6H_4$ | H | 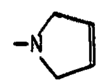 | |
| 201 | $3,4-(CH_3O)_2-C_6H_3$ | $4-Cl-C_6H_4$ | H | 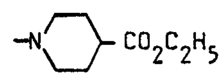 | |
| 202 | $3,4-(CH_3O)_2-C_6H_3$ | $4-Cl-C_6H_4$ | H | 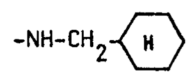 | |
| 203 | $3,4-(CH_3O)_2-C_6H_3$ | $4-Cl-C_6H_4$ | H | 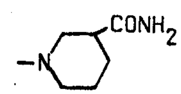 | |

| Verb. No. | A | B | $R^1$ | Q | physikalische Daten |
|---|---|---|---|---|---|
| 204 | $3,4-(CH_3O)_2-C_6H_3$ | $4-Cl-C_6H_4$ | H | 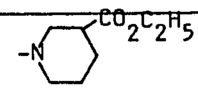 | |
| 205 | $3,4-(CH_3O)_2-C_6H_3$ | $4-ClC_6H_4$ | H | 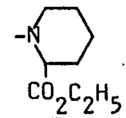 | |
| 206 | $3,4-(CH_3O)_2-C_6H_3$ | $4-Cl-C_6H_4$ | H | 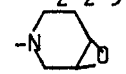 | |
| 207 | $3,4-(CH_3O)_2-C_6H_3$ | $4-Cl-C_6H_4$ | H | 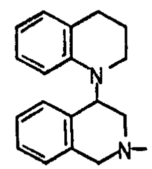 | |
| 208 | $3,4-(CH_3O)_2-C_6H_3$ | $4-Cl-C_6H_4$ | H | | |
| 209 | $3,4-(CH_3O)_2-C_6H_3$ | 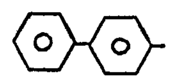 | H | 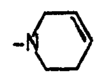 | |
| 210 | $3,4-(CH_3O)_2-C_6H_3$ | 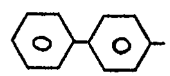 | H | 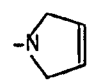 | |
| 211 | $3,4-(CH_3O)_2-C_6H_3$ | 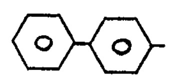 | H | 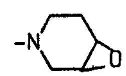 | |
| 212 | $3,4-(CH_3O)_2-C_6H_3$ | 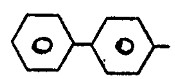 | H | 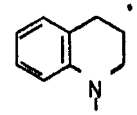 | |

Patentansprüche

1) Acrylsäureamide der Formel:

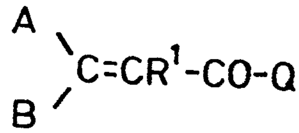

(I)

in der

$R^1$ für Wasserstoff, Halogen, Cyano, gegebenenfalls substituiertes Alkyl oder gegebenenfalls substituiertes Alkoxyalkyl steht

A für einen bis zu dreifach durch $R^2$ substituierten Phenylrest steht, wobei im Falle doppelter oder dreifacher Substitution $R^2$ unabhängig voneinander gewählt werden kann

B für

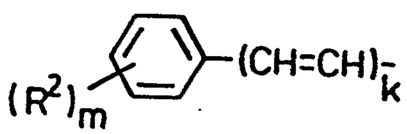

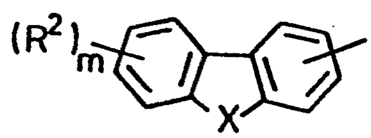

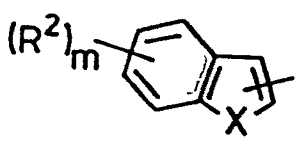

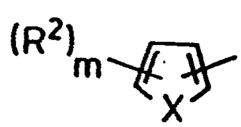

k für 0,1,2 steht

m für 0,1,2,3 steht, wobei $R^2$ unabhängig voneinander gewählt werden kann.

X für $CH_2$, O, S, NH oder N-Alkyl steht

$R^2$ für Halogen, Nitro, Hydroxy, Cyano, Carboxy, Alkoxycarbonyl, Carbamoyl, gegebenenfalls substituiertes Alkyl, gegebenenfalls substituiertes Alkoxy, gegebenenfalls substituiertes Alkyl-$S(O)_p$- mit p = 0,1,2, gegebenenfalls substituiertes Phenyl, gegebenenfalls substituiertes Phenoxy, Amino, Monoalkylamino, Dialkylamino, gegebenenfalls substituiertes Phenyl-$S(O)_p$ mit p = 0,1,2, gegebenenfalls substituiertes $C_3$-$C_7$-Cycloalkyl steht

Q für 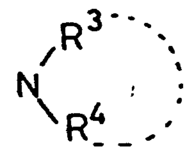 oder 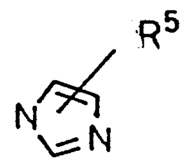

steht

$R^3$ für Wasserstoff, gegebenenfalls substituiertes Alkyl, gegebenenfalls substituiertes Phenyl steht

$R^4$ für substituiertes Alkyl, substituiertes $C_3$-$C_7$ Cycloalkyl, substituiertes Alkenyl, substituiertes Phenyl, gegebenenfalls substituiertes Alkinyl, gegebenenfalls substituiertes Alkoxy, gegebenenfalls substituiertes $C_5$-$C_{18}$-Alkyl

$(CH_2)_{(q+1)}-^6$ oder $(CH)-R^8-(CH_2)_q-CO-R^7$ (mit q = 0,1,2) steht

$R^6$ für gegebenenfalls substituiertes Alkoxy,
für gegebenenfalls substituiertes Alkylthio,
für gegebenenfalls substituiertes Alkylamino,
für gegebenenfalls substituiertes Dialkylamino,
für gegebenenfalls substituiertes Morpholino,
für gegebenenfalls substituiertes $C_3$-$C_7$-Cycloalkyl
für gegebenenfalls substituiertes $C_6$-$C_{10}$-Alkyl
für gegebenenfalls substituiertes Phenyl,
für gegebenenfalls substituiertes Dialkoxymethyl
für gegebenenfalls substituiertes Dialkylthiomethyl
für gegebenenfalls substituiertes 1,3-Dioxolan-2-yl

Halogen, Hydroxy, Amino, Mercapto steht

$R^7$ für Wasserstoff, Hydroxy, gegebenenfalls substituiertes Alkyl, Amino, Monoalkylamino, Dialkylamino, Alkoxy, gegebenenfalls substituiertes Morpholino, gegebenenfalls substituiertes Piperazino, gegebenenfalls substituiertes Imidazo, gegebenenfalls substituiertes Piperidino steht

$R^8$ für Wasserstoff, gegebenenfalls substituiertes Alkyl steht,

$R^3$ und $R^4$ gemeinsam für eine $C_3$-$C_5$-Alkylenkette, die durch O, NH, N-Alkyl, $S(O)_p$ mit p = 0,1,2 unterbrochen sein kann und ein oder mehrfach mit Halogen, Cyano, Nitro, Oxo, Hydroxy oder Mercapto substituiert ist, stehen.

$R^5$ für einen an ein beliebiges Kohlenstoffatom des Imidazol gebundenen Rest aus der Gruppe Wasserstoff, oder gegebenenfalls substituiertes Alkyl steht,

sowie gegebenenfalls die Salze der vorstehend definierten Verbindungen mit Säuren, Basen oder Komplexbildern.

2) Fungizides Mittel, enthaltend eine Verbindung nach Anspruch 1

3) Verfahren zur Bekämpfung von phytopathogenen Pilzen, dadurch gekennzeichnet, daß man eine fungizid wirksame Menge einer Verbindung der Formel I gemäß Anspruch 1 auf die Pilze oder durch Pilzbefall bedrohte Flächen, Pflanzen oder Saatgüter einwirken läßt.

4) Verfahren zur Herstellung von Verbindungen der Formel I

a) durch Umsetzung einer Acrylsäure der Formel

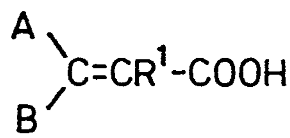

(II)

worin A, B und $R^1$ wie in Anspruch 1 definiert sind oder durch Umsetzung eines gegebenenfalls in situ hergestellten reaktionsfähigen Derivates von (II) mit einer Verbindung der Formel

HQ (III)

in der Q wie in Anspruch 1 definiert ist nach an sich bekannten Verfahren

oder

b) durch Umsetzung einer Acrylsäure der Formel (II) in der A, B und $R^1$ wie in Anspruch 1 definiert sind mit einem gewünschtenfalls susbstituierten Carbonyldiimidazol zu Verbindungen der Formel (I), in denen Q für ein gewünschtenfalls substituiertes Imidazolradikal steht,

c) durch Umsetzung eines Ketons der Formel (IV)

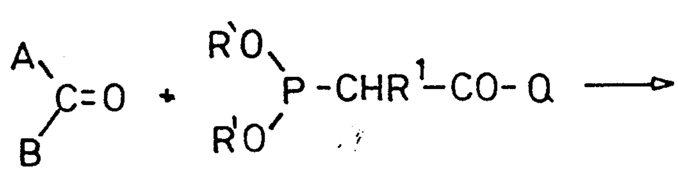

(I)

(IV) (XIV)

0208999

mit einem Phosphonoessigsäurederivat der Formel (XIV), wobei A, B, $R^1$ und Q wie in Anspruch 1 definiert sind und R' für eine niedere Alkylgruppe steht.

Europäisches Patentamt

# EUROPÄISCHER RECHERCHENBERICHT

Nummer der Anmeldung 0208999

EP 86109031.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | CHEMICAL ABSTRACTS, Band 71, Nr. 23, 8. Dezember 1969, Columbus, Ohio, USA<br>T. KURIHARA et al. "Local anesthetics. XXI. Derivatives of 3,3-disubstituted-3-hydroxypropionic acids"<br>Seite 358, Spalte 2, Seite 359, Spalte 1, Zusammenfassung-Nr. 112 757z<br>& Tohoku Yakka Daigaku Kenkyu Nempo 1967, No. 14,51-8<br>-- | 1,4 | C 07 C 103/58<br>C 07 C 83/10<br>C 07 C 121/43<br>C 07 C 143/78<br>C 07 C 147/107<br>C 07 C 147/14<br>C 07 C 149/41<br>C 07 D 211/34<br>C 07 D 211/44<br>C 07 D 211/70<br>C 07 D 207/20<br>C 07 D 233/56<br>C 07 D 263/08<br>C 07 D 295/18<br>C 07 D 307/54<br>C 07 D 307/91<br>C 07 D 317/28<br>C 07 D 233/24<br>C 07 D 333/60<br>C 07 D 333/76 |
| A | DE - A - 1 518 753 (ELI LILLY AND COMPANY)<br>* Ansprüche 1,6,9 *<br>-- | 1,4 | **RECHERCHIERTE SACHGEBIETE (Int. Cl.4)** |
| A | GB - A - 1 388 394 (MAKHTESHIM CHEMICAL WORKS LIMITED)<br>* Gesamt *<br>-- | 1-4 | C 07 C<br>C 07 D |
| A | EP - A2 - 0 143 142 (CIBA-GEIGY)<br>* Ansprüche 1,8 *<br>---- | 1,4 | |

Der vorliegende Recherchenbericht wurde für alle Patentansprüche erstellt.

| Recherchenort | Abschlußdatum der Recherche | Prüfer |
|---|---|---|
| WIEN | 15-10-1986 | HOFBAUER |

**KATEGORIE DER GENANNTEN DOKUMENTEN**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

Europäisches Patentamt

**EUROPÄISCHER RECHERCHENBERICHT**

0208999

Nummer der Anmeldung

EP 86109031.4

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| | | | A 61 N 37/18<br>A 01 N 43/00 |
| | | | RECHERCHIERTE SACHGEBIETE (Int. Cl.4) |
| | | | |

Der vorliegende Recherchenbericht wurde für alle Patentansprüche erstellt.

| Recherchenort | Abschlußdatum der Recherche | Prüfer |
|---|---|---|
| WIEN | 15-10-1986 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82